Europäisches Patentamt

European Patent Office

Office européen des brevets

(19)

(11) Publication number: **0 245 019**
**A2**

## EUROPEAN PATENT APPLICATION

(21) Application number: **87303817.8**

(22) Date of filing: **29.04.87**

(51) Int. Cl.⁴: **A 61 K 49/00**

(30) Priority: **30.04.86 US 857843**

(43) Date of publication of application: **11.11.87**
**Bulletin 87/46**

(84) Designated Contracting States: **AT BE CH DE ES FR GB GR IT LI LU NL SE**

(71) Applicant: **Davis, Michael A., 521 Hartford Street, Westwood Massachusetts 02090 (US)**
Applicant: **Raptopoulus, Vassilios, 110 Mill Street, Framingham Massachusetts 01701 (US)**

(72) Inventor: **Davis, Michael A., 521 Hartford Street, Westwood Massachusetts 02090 (US)**
Inventor: **Raptopoulus, Vassilios, 110 Mill Street, Framingham Massachusetts 01701 (US)**

(74) Representative: **Geering, Keith Edwin et al, REDDIE & GROSE 16 Theobalds Road, London WC1X 8PL (GB)**

(54) Low density contrast medium for diagnosis of pathologic conditions.

(57) A method for imaging the GI tract and the intestinal wall is described. The method comprises administering to a mammal a low density contrast medium comprising an oil in water emulsion containing from about 2 to about 50 percent by volume oil, and obtaining images of the GI tract and the intestinal wall. Also described is a low density contrast medium comprising an oil in water emulsion containing from about 2 to about 50 volume percent oil and a predetermined amount of soluble salts or sugars to maintain homeostasis in the intestine.

## LOW DENSITY CONTRAST MEDIUM
## FOR DIAGNOSIS OF PATHOLOGIC CONDITIONS

### Field of the Invention

The present invention relates to imaging body components, for example, by computed tomography (CT), ultrasonography (US) and magnetic resonance imaging (MRI), etc. to aid medical diagnosis of pathologic conditions of the body, and to contrast agents to aid in obtaining useful images of such body components, and particularly to low or negative density contrast agents to image and visualize the gastrointestinal (GI) tract and intestinal wall.

### Background of the Invention

Despite the recent technical advancements in medical imaging, there is no satisfactory method available to assess the wall of the GI tract. Although barium radiography and endoscopy are accurate and safe procedures, they can evaluate only the bowel lumen and mucosa. Computed tomography and, to a lesser extent, ultrasonography and magnetic resonance imaging best assess extraluminal abnormalities and assess only advanced mural pathology.

Untreated bowel infarction has a reported 90% mortality rate. Specific diagnosis is usually impossible on the basis of clinical findings alone. Plain film radiography and conventional contrast studies such as barium enema or GI series may provide significant information but the findings are usually non-specific. Angiography requires catheterization and, although it may identify an infarcted major intestinal artery, it has limited use for venous or incomplete arterial obstruction and small vessel occlusion. Absorption studies, radionuclide examinations and recently reported measurements of intraluminal pH provide indirect evidence of bowel ischemia with varying success. Magnetic resonance imaging of blood flow can be achieved, but this also is restricted to major vessels.

Although these methods provide diagnostic information about the presence or absence of bowel ischemia, the GI wall <u>per se</u> is not visualized or adequately assessed. Recent case reports suggest that this may be achieved with CT. See: Federle, et al., "Computed tomographic findings in bowel infarction," <u>AJR</u> <u>142</u>:91-95 (1984); Nichols, "Computed tomography in acute mesenteric vein thrombosis," <u>J. Comput. Assist. Tomogr.</u> <u>8</u>:171-172 (1984). Although thickened bowel wall, localized dilatation and intraluminal gas are cited as CT findings, the cases reported and our experience show that only extreme examples of bowel infarction have been diagnosed with this method by present techniques.

In abdominal CT scanning the need for preparation of the gastrointestinal tract with an oral contrast agent was addressed early on because unopacified bowel loops may be misinterpreted as soft tissue masses. Virtually every abdominal CT examination is done with a high density oral contrast agent in the form of either dilute water-soluble iodinated agents or barium

0245019

suspensions, and numerous schemes having been advocated for the optimal amount and timing of its administration. Still, one of the most common problems in interpreting abdominal CT scans is differentiating unopacified bowel loops from abdominal masses, because of poor mixing of the oral contrast agent with the intestinal contents. Furthermore, the wall of properly opacified normal bowel loops is rarely identified. Introduction of air in the gastrointestinal tract has recently been advocated. Although this method may identify the bowel loops, it requires wide CT density window settings, which are unsuitable for assessment of soft tissue densities such as the wall of the gastrointestinal tract.

The advantages and limitations of polyunsaturated fat, an oral negative contrast material, in outlining the stomach and duodenum in computed tomography were discussed by Baldwin in "Computed Tomography of the Pancreas: Negative Contrast Medium," Radiology 128:827-828 (1978). This medium was abandoned because of associated cramps and diarrhea and the readily available and safe high contrast agents.

The conventionally used high density oral contrast agents have limitations for bowel evaluation. In a retrospective review of over 400 abdominal scans performed at the University of Massachusetts Medical Center, optimal bowel opacification was achieved in only 63% of the cases. In 11% of the cases, additional views and "educated" clinical judgment were exercised. Furthermore, the bowel wall was rarely visualized. This is due to the fact that the normal bowel wall

0245019

is thin (5mm) and the high density of the
intraluminal high density contrast agent "overrides"
the soft tissue density of the wall (a phenomenon
due to technical factors, mainly partial volume
effect).

A method for obtaining useful images of the
intestinal wall is highly desirable. It would be
very useful for diagnosing various pathologic
conditions including, for example, intestinal
ischemia, neoplasia and imflammatory conditions
including Chrohn's disease, ulcerative colitis and
staging of tumors.

## Summary of the Invention

The present invention provides a method for
imaging and visualizing the intestinal wall. The
method comprises administering to a mammal a low
density contrast agent comprising an emulsion of an
oil in water, allowing sufficient time for the agent
to move through the intestine to fill the area to be
visualized, and forming an image of the intestine
including the wall. The image is preferably formed
using the techniques of computer tomography or
ultrasonography. The method of this invention not
only permits the visualizing of the intestinal wall
but also the evaluation of intestinal circulation
and perfusion to facilitate early diagnosis of
intestinal ischemia and objective assessment of the
degree of vascular insult. To evaluate circulation,
a high contrast agent is preferably administered
intravenously to enhance the image of the intestinal
wall.

The invention also provides a preferred low density contrast agent comprising an emulsion containing from about two to about fifty percent by volume oil in water and a predetermined amount of soluble salts and/or sugars to maintain homeostasis in the intestine.

Detailed Description of the Invention

In accord with the present invention consistent identification of bowel loops and visualization of the wall of the gastrointestinal tract of a mammal are obtained by prior administration of a low contrast agent comprising from about 2 to about 50 volume percent oil in water emulsion and scanning the GI tract with suitable imaging apparatus to form an image of the GI tract including the intestinal wall. Preferably, the contrast agent is administered orally. However, it may be administered by alternative means (e.g. via a nasogastric tube, intestinal tube, duo-tube, gastrostomy tube, as a rectal enema, etc.) in accord with the condition of the patient.

Various oils are suitable for preparing the low contrast agent used in accord with the present invention. Examples of such oils include corn oil, soybean oil, cottonseed oil, peanut oil, sunflower seed oil, safflower seed oil, and the like. Preferably, the oil is a polyunsaturated oil. Any suitable emulsifying agent, such as acacia gum, may be used to form the emulsion.

The low contrast medium is preferably administered in four doses at one hour intervals to provide complete filling of the intestine with the contrast medium. A quantity of about 4ml/kg of body

weight is administered for each dose for a total of 16ml/kg administered prior to imaging. This quantity may be adjusted by the physician as required for specific patients and specific circumstances.

As aforesaid, the concentration of oil in the emulsion of the contrast medium can be in the range of from about 2 to 50%. Preferably, the concentration of oil is in the range of from about 6 to about 25% and, most preferably, is about 12.5%.

Scanning is performed after filling the GI tract with the contrast medium. When scanning by CT, typically 10mm in thickness cross sectional scans through the abdomen are made sequentially until the whole GI tract is scanned.

To evaluate blood circulation and blood perfusion to the intestine to determine areas of ischemia, dynamic scanning is performed, typically one scan every two seconds at a single level. After the GI tract is filled with the low contrast medium as discussed above, a bolus of high contrast medium (about 0.4ml/kg) is injected intravenously. Meglumine diatrizoate 60% (Reno-M-60$^{TM}$ available from Squibb), or the like, can be used for the intravenous high contrast agent. Scanning is begun prior to injection of the high contrast medium to obtain one complete scan prior to injection and the scanning is continued to obtain about 20 sequential scans at one cross sectional level. The process is then repeated at a second cross sectional level until all regions of interest are scanned. Time activity curves are compared with those obtained from the aorta, inferior vena cava and other selected tissues. The dynamic scanning is followed by conventional scanning with 10mm thick cross sectional slices of the abdomen to obtain contrast enhanced images of the GI wall throughout its entire length.

Intestinal vasodilating agents can be administered to enhance the differences between normal and ischemic GI wall.

The present invention will be further illustrated by the following examples.

Example 1

One liter of stock low contrast medium consisting of a 50% corn oil emulsion is prepared as follows.

Ingredients and Equipment Required:

        Blender
        1000ml graduate
        Acacia gum - 100gms
        Corn oil - 500ml
        Sterile Water for Irrigation to 1000ml
        Alcohol 1000ml
        Acetone - 60ml
        Hand Homogenizer

Place alcohol into the blender container. Blend for 2 minutes on low speed. Let alcohol stand in blender for 20 minutes. Empty alcohol out of blender and discard. Place acetone in blender and tilt container so that it covers all surfaces. Discard acetone and let air dry for 5 minutes.

Place acacia gum into blender and add around 60ml of corn oil. Blend on high speed to form a thick muscilage. Continue blending for 2 minutes then add remaining corn oil and blend for an additional 5 minutes. Add 100ml of sterile water then blend for 15 minutes. Add sterile water to approximately 900ml. Transfer to graduate and add sterile water to 1000ml. Transfer back to blender and blend for an additional 15 minutes. Transfer to hand homogenizer and pass emulsion through homogenizer.

Preferably, instead of sterile water, a solution of sodium chloride and sugar in the sterile water is used to prepare the low density contrast agent of the present invention. Typically, a solution containing 5 or 10% dextrose and normal or one-half normal physiological saline is used to maintain fluid balance in the intestines. It is presently preferred to use a 5% dextrose and one-half normal physiological saline solution (i.e. $D_5$-1/2NS) to prepare the low density contrast agent.

The 50% corn oil emulsion can be further diluted with sterile water to obtain contrast medium having lower oil concentration. Preferably, the diluted emulsion is passed through the hand homogenizer.

Example 2

The 50% corn oil emulsion prepared in Example 1 was used as a low density oral contrast medium for abdominal CT scanning of eight dogs weighing 20-35 Kgs, four rabbits weighing 2-4 Kgs, and two piglets weighing 2 Kgs. Originally, the 50% corn oil emulsion was tested. Subsequently, 25%, 12.5%, 6.25% and 3.125% emulsions were tested by diluting the 50% emulsion. The animals were also scanned prior to and after preparation of their

0245019

gastrointestinal tract with a conventional high density oral contrast material (2% solution of diatrizoate sodium), water and air.

The animals were anesthetized with 0.7 mg/kg Nembutal, intubated, and a nasogastric tube was inserted. In the dogs, a total of 400 ml of the oral low contrast medium of Example 1 was administered prior to scanning, divided into four hourly doses of 100 ml each. The rabbits and piglets received a total of 100 ml of the oral low contrast medium. In the first six examinations performed in dogs, a total of 800 ml of oral contrast was administered but subsequently 400 ml appeared to be an adequate amount. For all animals the same CT scanner was utilized as used for the humans.

For each examination three sets of scans were performed: the first without intravenous administration of iodinated contrast; the second during intravenous administration of a bolus of 2 ml/kg of 60% meglumine diatrizoate (Reno-M-60$^{TM}$, Squibb, Princeton, NJ), injected at a rate of 1 ml/sec while rapid sequence scanning with table incrementation was performed; and a third set of scans was performed after the bolus injection. In three dogs, instead of the second set of scans, rapid sequence (dynamic) scanning over one cross sectional level was performed during intravenous administration of the iodine solution.

Thirty-two abdominal scan series were performed in the animals, each one of which constituted three separate sets (before, during and after intravenous bolus), totalling 96, scans which were evaluated in respect to bowel loop identification and intestinal wall visualization.

There was consistent identification of the bowel loops and visualization of the wall of the gastrointestinal tract with the use of the 50%, 25% and 12.5% corn oil emulsions. This was observed both before, during and after administration of iodinated contrast material. The normal bowel wall thickness in the dog measured 3.5 to 4mm. With the lower concentrations of 6.25% and 3.125% corn oil emulsions, bowel loop identification was inconsistent and bowel wall visualization could be achieved only with intravenous administration of high density contrast medium. With the exception of mild diarrhea observed with the 50% concentration only, no other clinical ill-effects were observed in the dogs. Since both the 50% and the 25% corn oil emulsions showed no significant advantage in bowel loop identification and wall visualization over the 12.5% emulsion, the latter concentration was considered the optimal low density contrast material for our animal model.

The use of low density contrast medium in accord with the present invention proved to be superior to conventional regimens that use high contrast medium. For example, with the conventional 2% high density contrast solution of diatrizoate sodium, inconsistent mixing of the contrast medium with the intestinal contents was observed resulting in numerous unopacified bowel loops. Furthermore, the intestinal wall of the opacified loops was rarely visualized. Both these parameters were poor when water or no oral preparation were used. Air used as the oral contrast medium had similar problems as in humans. Although the gastrointestinal tract was well identified, for visualization of the bowel wall wide CT window settings were needed resulting in suboptimal soft tissue evaluation. Finally, both rabbits and piglets were not as satisfactory as dogs for good CT evaluation of their gastrointestinal tract due to their small size.

The optimal oral contrast preparation for imaging of the gastrointestinal wall of dogs consists of 400 ml of a 12.5% corn oil emulsion administered in four hourly divided doses. With this regimen the intraluminal gastrointestinal contents had a CT density ranging from -20 to +20 units. This provided an excellent contrast differentiation with the bowel wall which has a density of soft tissue, ranging from +30 to +55 units. Administration of intravenous iodine solution further increased the density of the gastrointestinal wall to up to +100 units further enhancing this contrast differentiation while still remaining within optimal density differentiation for CT scanning. The extra-intestinal peritoneal fat having a CT density ranging from -90 to -5 units served as an endogenous contrast medium facilitating the visualization of the intestinal wall. Thus, the bowel wall could be clearly visualized. Dynamic scanning over one cross sectional level, performed in three dogs, provided measurable changes in bowel wall density reflecting vascular perfusion.

Example 3

Adult mongrel dogs of approximately 25 kg weight are scanned with a GE 8800 Scanner or a GE 9800 Scanner 24 hours before, and 24 hours and 48 hours after surgical induction of intestinal ischemia. They are then euthanised and gross inspection and histologic examination of the bowel is performed.

Intestinal ischemia is induced in dogs in the manner described by Marsden et al Gut 10: 121-130 (1969). After induction of anesthesia, intestinal ischemia is induced by ligation of the control mesenteric and marginal arteries and control mesenteric marginal and common colic arteries. In another group of dogs, the corresponding veins are ligated. Indirect measurements of intramural pH are made. These provide a measure of the adequacy of tissue oxygenation of the intact dog intestine. In order to obtain sequential evaluations, the tonometer is brought out subcutaneously and up through the scapula together with a second tube for infusion of low density contrast material.

CT scanning is performed after filling the GI tract with 400 ml 12.5% corn oil emulsion via a nasogastric tube (pre-ischemia) and via the intestinal tube inserted at surgery. 10mm in thickness cross sectional CT scans through the abdomen are obtained. Two sections with optimal bowel loop preparation are selected. Sequential, dynamic scanning is performed (one every 2 seconds) at a single level, while injecting intravenously 20 ml (0.4ml/kg) of Hypaque 60[TM] (Winthrop, New York) at a rate of 1 ml/sec using a power injector. Scanning is started two seconds prior to initiation of the injection. 20 sequential scans are taken in 40 seconds, providing images before during and after the intravenous contrast injection. The process is repeated at a second cross sectional level. Time activity curves are generated from regions of interest on the bowel wall and compared with those obtained from the aorta, inferior vena cava, liver, spleen, kidneys and muscle. Dynamic scanning is followed by conventional CT scanning with 10mm thick cross sectional slices of the abdomen in order to obtain contrast enhanced images of the GI wall throughout its length.

One of the major problems in interpreting time-density curves thus obtained is that they are influenced by a number of factors. Some of these factors include the dose of intravenous contrast material used, the rate of infusion, the level of resting blood supply to the bowel, the timing of the scan sequence and the size of the region of interest utilized, to mention only a few. To overcome these problems we decided to make another measurement during maximum vasodilatation induced by dipyridamole. This approach enhances sensitivity by maximizing the difference between normally and abnormally perfused tissues. The suitably of dipyridamole, in the case of the bowel, is based on the fact that besides the coronaries, the only other circulation in which the agent causes vasodilation is the splanchnic. In four dogs, the time density activity curves showed dramatically different patterns between resting (smooth bell shaped curve) and after dipyridamole (regularly alternating high peaks and valleys).

Example 4

Example 3 is repeated except ultrasonography using a real-time ultrasound scanner is performed instead of CT. Images of the bowel are obtained and analyzed with respect to mucosal detail, bowel wall thickness and serosal detail. Comparisons are made of the US images pre and post ischemia and pre and post dipyridamole. In addition, deep pulse doppler measurement of the GI wall is obtained pre and post ischemia and pre and post pharmacologic vasodilation with the dipyridamole.

Example 5

A dog is given 500-750 ml low density contrast agent (containing 12.5% corn oil prepared in accord with Example 1 using $D_5$-1/2 NS instead of sterile water) by a rectal enema and CT of the large bowel is performed in a manner similar to that described in Ex. 2. It is preferable to cleanse the colon of fecal material with a standard cathartic preparation prior to rectal administration of the low contrast agent, but this is not essential. The large intestine is visualized from cecum to rectum and on occasion the distal ileum can be visualized when the contrast agent is refluxed through the ileocecal valve into the terminal ileum. The intestine wall is consistently visualized superior to images obtained with barium, sodium diatrozate, or air.

The present invention has been described in detail including the preferred embodiments thereof. However, it will be appreciated that those skilled in the art, upon consideration of the disclosure herein, may make modifications and improvements within the spirit and scope of the invention as defined by the claims.

0245019

C L A I M S :

1. A low density contrast medium comprising an oil in water emulsion containing from about 2 to about 50 volume percent oil and a predetermined amount of soluble salts or sugars to maintain homeostasis in the intestine.

2. A contrast medium according to claim 1 wherein said medium contains sodium chloride and dextrose.

3. A contrast medium according to claim 2 wherein said medium contains 5% by weight dextrose and sufficient sodium chloride to make normal physiological saline.

4. A contrast medium according to claim 2 wherein said medium contains 10% be weight dextrose and sufficient sodium chloride to make normal physiological saline.

5. A contrast medium according to claim 2 wherein said medium contains 5% dextrose and sufficient sodium chloride to make one-half normal physiological saline.

6. A method for evaluating intestinal blood circulation or perfusion in a mammal, said method comprising filling the gastrointestinal tract of said mammal with a low contrast medium comprising an oil in water emulsion containing from about 2 to about 50 volume percent oil, and obtaining deep pulse doppler measurements of the intestinal wall.

0245019

7.        A method for imaging the intestinal wall, said method comprising administering to a mammal a low density contrast medium comprising an oil in water emulsion containing from about 2 to about 50 percent by volume oil, and obtaining an image of the intestinal wall.


8.        A method for evaluating intestinal blood circulation or perfusion in a mammal, said method comprising:

(1)    filling the gastrointestinal tract with a low contrast medium comprising an oil in water emulsion containing from about 2 to about 50 volume percent oil;

(2)    dynamically scanning said gastrointestinal tract to obtain sequential images of the intestinal wall; and

(3)    intravenously injecting a high contrast medium into the mammal to enhance the contrast of the blood vessels in the intestine.